# EUROPEAN PATENT APPLICATION

(11) **EP 2 392 373 A1**
(43) Date of publication of application: **07.12.2011**
(21) Application number: 11250576.3
(22) Date of filing: 02.06.2011
(51) Int. Cl.: A61M 15/08, B05B 11/02

(54) **Nasal spray device**

(30) Priority: 04.06.2010 TW 099210663
(71) Applicant: Su, Cheng-Yuan, Hsin-Chu (TW)
(72) Inventor: Su, Cheng-Yuan, Hsin-Chu (TW)
(74) Representative: Brown, Michael Stanley

(57) **Abstract**

A nasal spray device is an environment friendly spray device so as to spray the liquid medicine in the container from the outlet. The nasal spray device includes a push member, a mount, a rod, a block, a resilient member and a cylindrical body. The resilient member made by metal is received in the cylindrical body so that it does not contact the liquid medicine which is prevented from being spoiled. The cylindrical body does not have any bead received therein so as to reduce the number of parts.

## Description

### BACKGROUND OF THE INVENTION

### 1. FIELD OF THE INVENTION

The present invention relates to a spray device, and more particularly, to a nasal spray device for injecting liquid medication into the user's nose.

### 2. BACKGROUND OF THE INVENTION

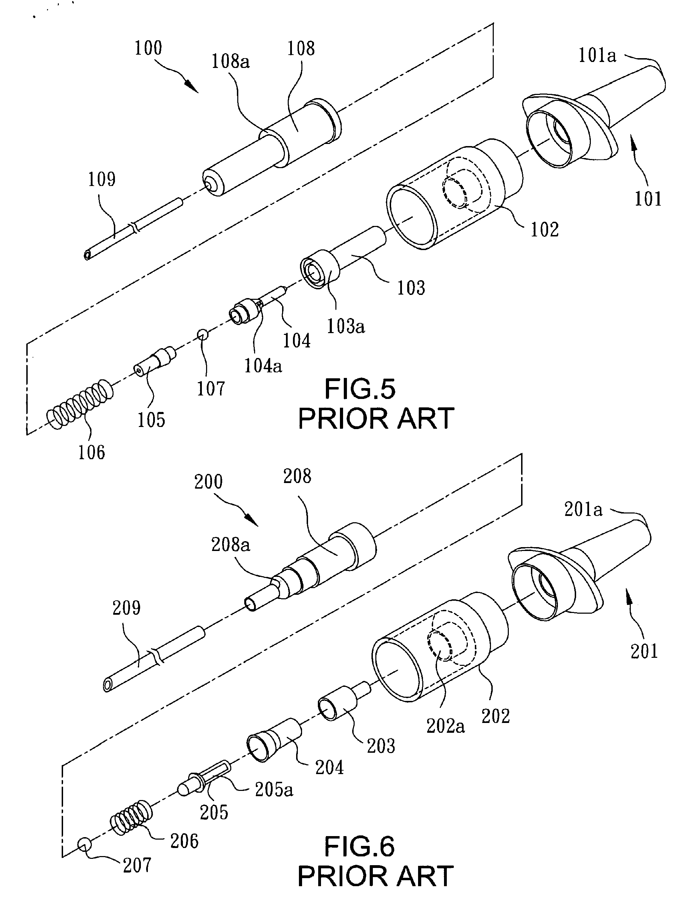

Many people are suffered by allergy due to air pollution and one of the symptoms is itchy nose so that the nasal spray device is developed to deliver medicine into the nose to let the patients feel comfortable. The convention nasal spray device 100 is shown in Fig. 5 and generally includes a push member 101 with an outlet 101 a and a mount 102 is connected to the push member 101. The mount 102 has a suction tube 103 connected thereto which has an annular valve 103a connected thereto. A cone-shaped tube 104 is connected to the annular valve 103a of the suction tube 103. A bead 107 is located in the cone-shaped tube 104 which has a hole 104a sealed by the bead 107. A tube 105 is connected to the cone-shaped tube 104 and a spring 106 is mounted to the tube 105. A reception tube 108 accommodates the suction tube 103, the cone-shaped tube 104, the tube 105 and the spring 106 therein in sequence. A shoulder 108a is formed on the reception tube 108 and a hose 109 is mounted to the reception tube 108 and stopped by the shoulder 108a. The mount 102 is connected to a container which is not shown so as to suck the liquid medicine and spray via the outlet 101 a.

By pushing the push member 101 to let the air in the container escape from the outlet 101 a and the lower pressure sucks the liquid medicine in the container into the reception tube 108 via the hose 109. The annular valve 103a of the suction tube 103 moves downward along the reception tube 108 and a seal status is formed when the outer periphery of the annular valve 103 contacts the shoulder 108a. Therefore, the liquid medicine in the reception tube 108 is pressed and flows upward via the hole 104a and released from the outlet 101 a of the push member 101. When the push member 101 is released, the liquid medicine and air are back to the reception tube 108 via the hose 109.

Another conventional nasal spray device 200 is disclosed in Fig. 6 and generally includes a push member 201 with an outlet 201 a and a mount 202 is connected to the push member 201. The mount 202 has a suction tube 203 which extends through the inner tube 202a in the mount 202 and is connected to the push member 201. A sleeve 204 is connected to the other end of the suction tube 203 and a rod 205 is inserted into the sleeve 204. The rod 205 has paths 205a defined in the outer periphery thereof and a spring 206 is mounted to the rod 205. A bead 207 is located at a distal end of the spring 206 and located in a reception tube 208 which has multiple sections with reduced diameters and a shoulder 208a is formed on the reception tube 208. A hose 209 is connected to the reception tube 208. The assembly is connected to a container (not shown) by the mount 202 so as to suck the liquid medicine and spray via the outlet 201 a.

By pushing the push member 201 to let the air in the container escape from the outlet 201 a and the lower pressure sucks the liquid medicine in the container into the reception tube 208 via the hose 209. The spring 206 moves the rod 205 to press the bead 207 which seals the shoulder 208a of the reception tube 208 and a gap is defined between the rod 205 and the sleeve 204. The liquid medicine in the reception tube 208 flows into the suction tube 203 via the paths 205a and is injected from the outlet 201 a of the push member 201. When the push member 201 is released, the liquid medicine and air are back to the reception tube 208 via the hose 209.

Although the conventional push-type nasal sprays 100, 200 can suck the liquid medicine and spray the liquid medicine from the outlet, the spring 106, 206 and the bead 107, 207 are made by metal and in contact with the liquid directly and may cause chemical change to the liquid medicine and/or be harmful to the users. Besides, the spoiled liquid medicine is difficult to be recycled and may pollute the environment. Furthermore, the conventional nasal spray devices include too many parts and this makes them to be costly.

The present invention intends to provide a nasal spray device which improves the shortcomings of the conventional nasal spray devices.

### SUMMARY OF THE INVENTION

The present invention relates to a nasal spray device and comprises a push member having an outlet from which the liquid medicine is sprayed. A tubular portion is connected to a lower end of the push member. A mount is connected to a container and has a reception portion connected with the push member. A passage is defined centrally through the mount and the tubular portion extends through the passage. A rod has a guide tube on a first end thereof and the guide tube extends through the tubular portion. A second end of the rod has an insertion and multiple paths are defined axially in the outer surface thereof. A flange extends radially outward from the rod and is located between the insertion and the guide tube. A protrusion is connected to a side of the flange. A resilient member is mounted between the tubular portion of the push member and the mount. A block is movably mounted to the first end of the rod and has a reception tube so that the guide tube extends through the reception tube. A central hole is defined between the reception tube and the guide tube. The protrusion is engaged with the reception tube to seal the reception tube. The resilient member moves the rod back and forth. A cylindrical body is a tubular member with gradually reduced diameter and has a top section and a bottom section whose diameter is smaller than that of the top section. The top section has a barrel in which the block and the rod are accommodated. A tightening valve is formed between the top and bottom sections and the rod snugly extends through the tightening valve. By pushing the push member, the liquid medicine in the container is sucked and sprayed from the outlet.

The primary object of the present invention is to provide a nasal spray device wherein the resilient member made by metal is received in the cylindrical body and does not contact the liquid medicine which is prevented from being spoiled.

Another object of the present invention is to provide a nasal spray device wherein the cylindrical body does not have any bead received therein to reduce the number of parts.

The present invention will become more obvious from the following description when taken in connection with the accompanying drawings which show, for purposes of illustration only, a preferred embodiment in accordance with the present invention.

### BRIEF DESCRIPTION OF THE DRAWINGS

Fig. 1 is an exploded view to show the nasal spray device of the present invention;
Fig. 2 is a cross sectional view of the nasal spray device of the present invention;
Fig. 3 is a cross sectional view to show that the push member of the nasal spray device of the present invention is pushed downward;
Fig. 4 is a cross sectional view to show that the push member of the nasal spray device of the present invention is released;
Fig. 5 is an exploded view to show a conventional nasal spray device, and
Fig. 6 is an exploded view to show another conventional nasal spray device.

### DETAILED DESCRIPTION OF THE PREFERRED EMBODIMENT

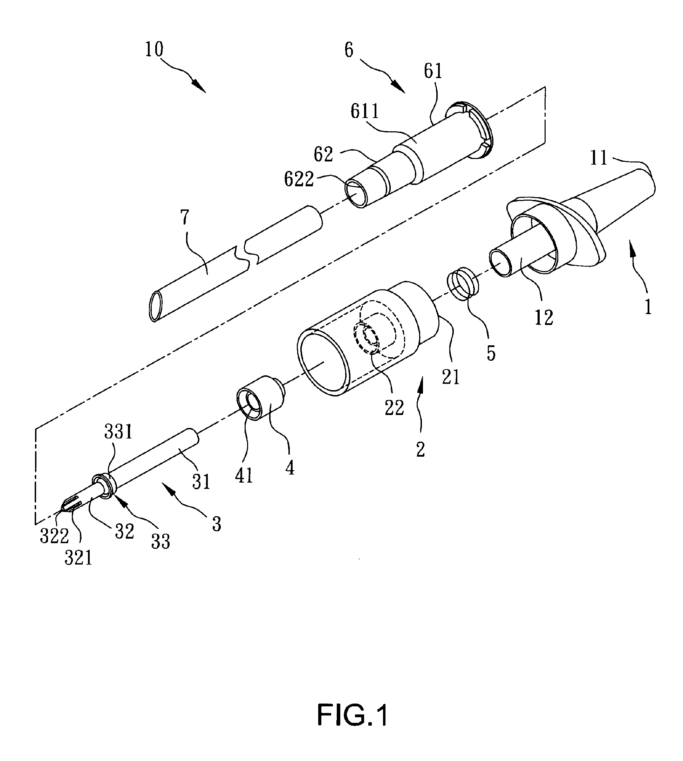

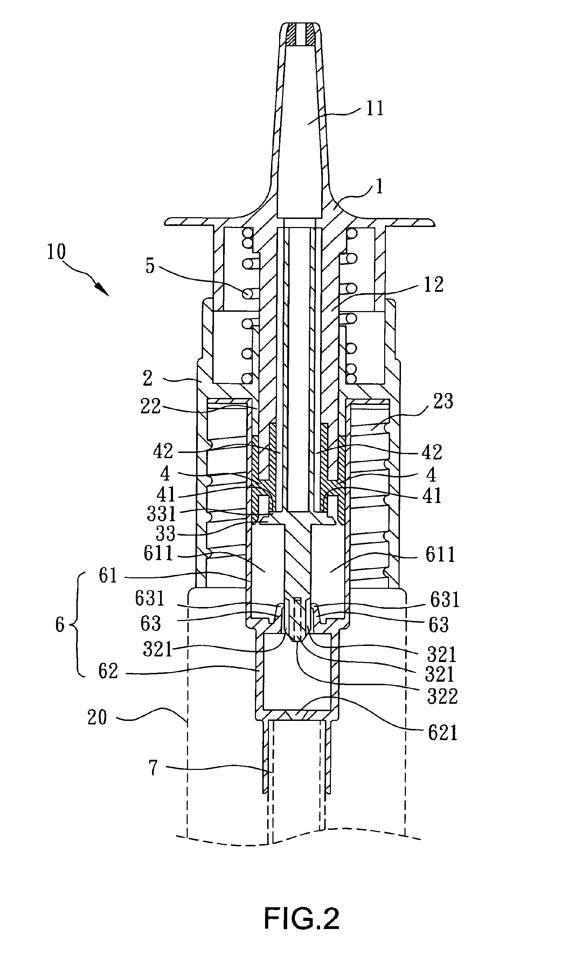

Referring to Figs. 1 and 2, the nasal spray device of the present invention comprises a push member 1, a mount 2, a rod 3, a block 4, a resilient member 5 and a cylindrical body 6, wherein the push member 1 has an outlet 11 defined therein and a tubular portion 12 is connected to a lower end of the push member 1. The mount 2 is to be connected to a container 20 and has a reception portion 21 connected with the push member 1 and a passage 22 is defined centrally through the mount 2. The tubular portion 12 extends through the passage 22. The rod 3 has a guide tube 31 on the first end thereof and the guide tube 31 extends through the tubular portion 12. The second end of the rod 3 has an insertion 32 and multiple paths 321 are defined axially in the outer surface thereof. A flange 33 extends radially outward from the rod 3 and is located between the insertion 32 and the guide tube 31. A protrusion 331 is connected to a side of the flange 33.

The resilient member 5 is mounted between the tubular portion 12 of the push member 1 and the mount 2 so as to provide a force to move the push member 1 and the rod 3 back. The block 4 is movably mounted to the first end of the rod 3 and has a reception tube 41 so that the guide tube 31 extends through the reception tube 41. A central hole 42 is defined between the reception tube 41 and the guide tube 31. The protrusion 331 is engaged with the reception tube 41 to seal the reception tube 41. The resilient member 5 moves the rod 3 back and forth. The cylindrical body 6 is a tubular member with gradually reduced diameter and includes a top section 61 and a bottom section 62 whose diameter is smaller than that of the top section 61. The top section 61 has a barrel 611 in which the block 4 and the rod 3 are accommodated. A tightening valve 63 is formed between the top and bottom sections 61, 62 and the rod 3 snugly extends through the tightening valve 63. The cylindrical body 6 has an entrance 621 defined in the lower end thereof and the entrance 621 is located corresponding to the distal end 322. The cylindrical body 6 has a connection hole 622 is defined in the lower end thereof so as to be connected with a hose 7 which is inserted into the container 20. The tightening valve 63 includes a tightening end 631 which defines an opening at a distal end of the tightening valve 63. The tightening end 631 is resiliently mounted to the insertion 32 of the rod 3. After the parts mentioned above are assembled, the mount 2 is connected to the container 20.

The block 4 is movably mounted to the rod 3 to form opened or closed status by the pressure. When in opened status, a gap 8 is defined between the reception tube 41 and the protrusion 331, so that the liquid medicine in the container 20 flows into the tubular portion 12 via the gap 8 and the central hole 42 and is sprayed from the outlet 11 by pushing the push member 1.

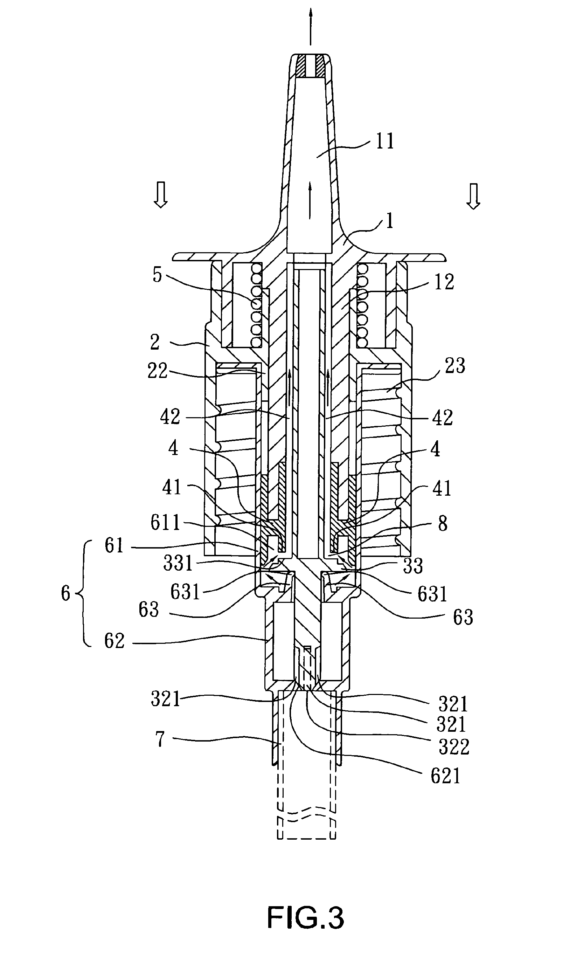

Further referring to Fig. 3, when the push member 1 is pushed, the rod 3 is lowered by the pressure and the space in the bottom section 62 is compressed until the distal end 322 of the rod 3 moves downward to seal the entrance 621 of the cylindrical body 6. The tightening end 631 contacts the upper portion of the insertion 32 to form a sealed status of the cylindrical body 6. In the meanwhile, the protrusion 331 on the flange 33 is separated from the block to form the gap 8, and the liquid medicine quickly passes through the central hole 42 and the tubular portion 12 and sprayed from the outlet 11.

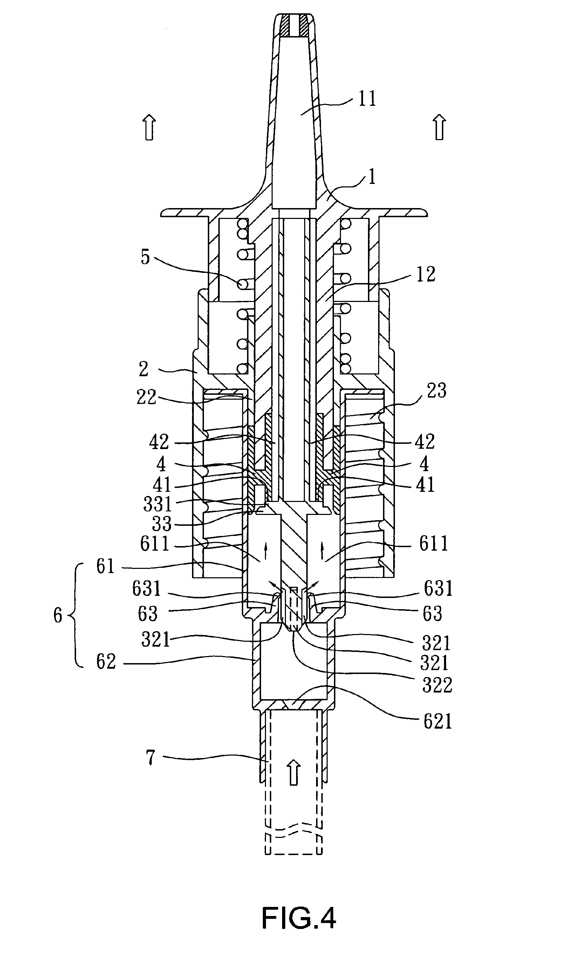

Referring to Fig. 4, when the push member 1 is released, the resilient member 5 pushes the rod 3 and the block 4 upward, and the protrusion 331 on the flange 33 is matched with the block again. The distal end 322 of the rod 3 is removed from the entrance 621 of the cylindrical body 6 and the tightening end 631 only contacts the lower end of the paths 321 so that the paths 321 are opened. Because the press in the container 20 suddenly increases, the liquid medicine is sucked into the barrel 611 via the entrance 621 and the paths 321.

The present invention includes the following advantages which are:
1. The nasal spray device is composed limited number of parts so as to be manufactured and assembled at low cost and the time required is reduced.
2. The resilient member 5 is located between the tubular portion
3. 12 and the mount 2, and the tightening end 631 and the insertion 32 replace the bead so that the liquid medicine in the container 20 is not in contact with metal part and prevented from being spoiled.

## Claims

1. A nasal spray device comprising:
a push member having an outlet defined therein and a tubular portion connected to a lower end of the push member;
a mount adapted to be connected to a container, the mount having a reception portion connected with the push member and a passage defined centrally through the mount, the tubular portion extending through the passage;
a rod having a guide tube on a first end thereof and the guide tube extending through the tubular portion, a second end of the rod having an insertion and multiple paths defined axially in an outer surface thereof, a flange extending radially outward from the rod and located between the insertion and the guide tube, a protrusion connected to a side of the flange;
a resilient member mounted between the tubular portion of the push member and the mount;
a block movably mounted to the first end of the rod and having a reception tube so that the guide tube extends through the reception tube, a central hole defined between the reception tube and the guide tube, the protrusion engaged with the reception tube to seal the reception tube, the resilient member moving the rod back and forth, and
a cylindrical body being a tubular member with gradually reduced diameter and having a top section and a bottom section whose diameter is smaller than that of the top section, the top section having a barrel in which the block and the rod are accommodated, a tightening valve formed between the top and bottom sections and the rod snugly extending through the tightening valve.

2. The device as claimed in claim 1, wherein the mount has a threaded portion which is adapted to be connected with the container.

3. The device as claimed in claim 1, wherein a gap is defined between the reception tube and the protrusion.

4. The device as claimed in claim 1, wherein the cylindrical body has a connection hole defined in a lower end thereof.

5. The device as claimed in claim 4, wherein the connection hole is connected with a hose which is adapted to insert into the container.

6. The device as claimed in claim 1, wherein the tightening valve includes a tightening end which defines an opening at a distal end of the tightening valve, the tightening end is resiliently mounted to the insertion of the rod.
